(19) Europäisches Patentamt
European Patent Office
Office européen des brevets

(11) Publication number: **0 144 441 A1**

# (12) EUROPEAN PATENT APPLICATION

published in accordance with Art. 158(3) EPC

(21) Application number: **84902059.9**

(51) Int. Cl.[4]: **A 61 K 31/425**

(22) Date of filing: **21.05.84**

Data of the international application taken as a basis:

(86) International application number:
**PCT/JP84/00255**

(87) International publication number:
**WO84/04678 (06.12.84 84/28)**

(30) Priority: **23.05.83 JP 90364/83**

(43) Date of publication of application:
**19.06.85 Bulletin 85/25**

(84) Designated Contracting States:
**BE FR**

(71) Applicant: **EARTH CHEMICAL CO., LTD.**
**3218-12, Sakoshi**
**Ako-shi Hyogo 678-01(JP)**

(72) Inventor: **YAMAMOTO, Itaru**
**1-102, Kikyo-machi Hanajiri Okayama-shi**
**Okayama 701-01(JP)**

(74) Representative: **Türk, Dietmar, Dr. rer. nat. et al,**
**Redies, Redies, Türk & Gille Patentanwälte**
**Brucknerstrasse 20**
**D-4000 Düsseldorf 13(DE)**

## (54) BLOOD PRESSURE-CONTROLLING AGENT.

(57) A blood pressure-controlling agent containing as an effective ingredient a trithiazolepentamethine cyanine compound represented by general formula (I):

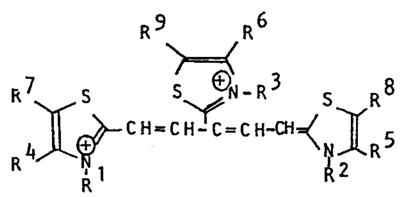

$2X^{\ominus}$ (1)

(wherein $R^1$, $R^2$, and $R^3$ may be the same or different and each represents an alkyl group containing 1 to 15 carbon atoms, $R^4$, $R^5$, $R^6$, $R^7$, $R^8$, and $R^9$ may be the same or different and each represents a hydrogen atom, an alkyl group containing 1 to 15 carbon atoms, a hydroxy group or a halogen atom, and X represents a halogen atom, a perchlorate residue, a nitrate residue or an organic acid residue). This agent shows no toxicity nor side effects, and is useful for prophylaxis and treatment of diseases due to hypertention in general.

EP 0 144 441 A1

./...

FIG. 1

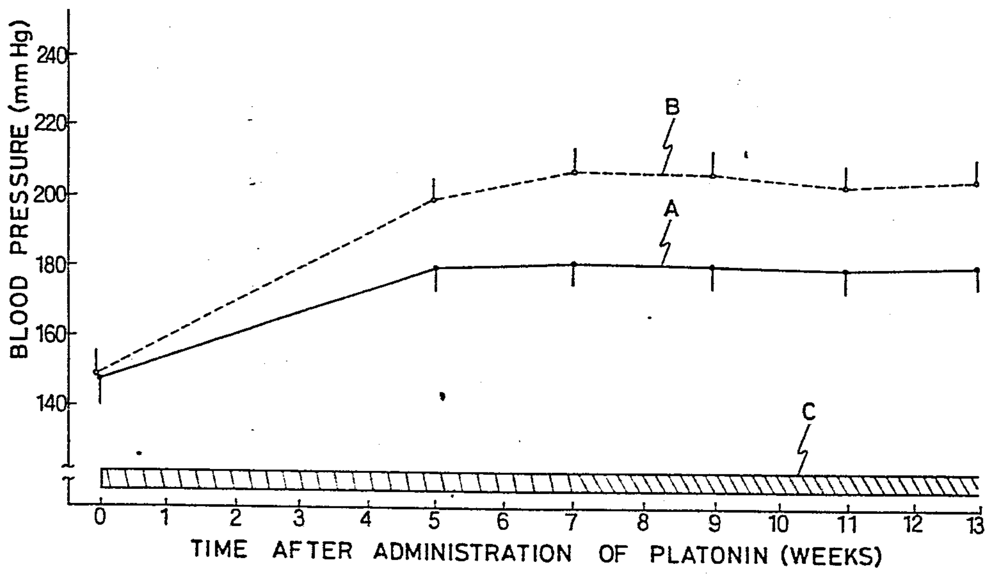

BLOOD PRESSURE (mm Hg)
240
220
200
180
160
140
B
A
C
0
1
2
3
4
5
6
7
8
9
10
11
12
13
TIME AFTER ADMINISTRATION OF PLATONIN (WEEKS)

0144441



TITLE MODIFIED
see front page

DESCRIPTION

# BLOOD PRESSURE-MODULATING AGENT

## TECHNICAL FIELD

The present invention relates to a novel blood pressure-modulating agent.

## BACKGROUND ART

Heretofore, for instance, thiazide hypotensive diuretics, vasodilators, β-adrenoreceptor blocking agents, α-adrenoreceptor blocking agents, etc. have been used as a medicament for hypertension. However, when these medicaments are administered to patients for a long-term period, they suffer from side effects such as orthostatic hypotension, bradycardia or tachycardia, vomiting, nausea, headache and dizziness. Thus, it is the present situation that sufficiently satisfactory medicaments are few.

An object of the present invention is to provide a blood pressure-modulating agent which is nontoxic and causes no side effects.

## DISCLOSURE OF THE INVENTION

The present invention provides a blood pressure-modulating agent whcih contains as an effective ingredient a trithiazole pentamethine cyanine compound represented by the general formula (I):

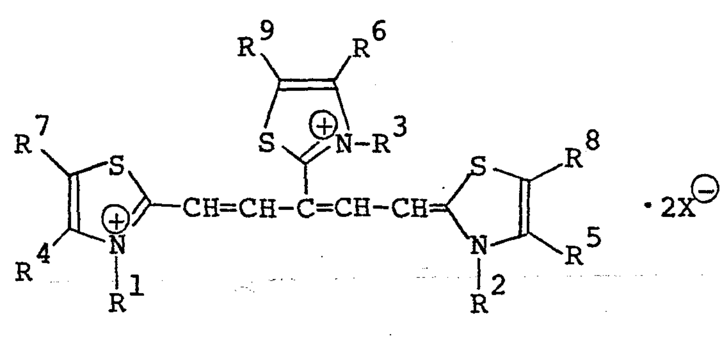

(I)

wherein $R^1$, $R^2$ and $R^3$ are the same or different and each

is an alkyl group having 1 to 15 carbon atoms, $R^4$, $R^5$, $R^6$, $R^7$, $R^8$ and $R^9$ are the same or different and each is hydrogen atom, an alkyl group having 1 to 15 carbon atoms, hydroxyl group or a halogen atom, and X is a halogen atom, the residual group of perchloric acid, the residual group of nitric acid or the residual group of an organic acid.

The trithiazole pentamethine cyanine compound represented by the above-mentioned general formula (I) is effective as a blood pressure-modulating agent which is nontoxic and causes no side effects.

Among the compounds represented by the general formula (I), the compound wherein $R^1$, $R^2$ and $R^3$ are n-heptyl group, $R^4$, $R^5$ and $R^6$ are methyl group, $R^7$, $R^8$ and $R^9$ are hydrogen atom, and X is iodine is a known compound called platonin.

## BRIEF DESCRIPTION OF THE DRAWINGS

Fig. 1 is a graph showing the effect of platonin on blood pressure in SHR.

Fig. 2 is a graph showing the effect of platonin on body weight in SHR.

Fig. 3 is a graph showing the effect of platonin on blood pressure in normal rats.

The above-mentioned platonin has been known to have pharmacological actions such as antibacterial action, acceleration of wound healing, stimulation of reticular hematopoietic organ and endocrine organ and enhancement of antibody formation.

However, it has so far been unknown that the compounds (I) including platonin neither have toxicity nor cause side effects and exert an excellent antihypertensive effect, in particular, when given orally. These findings have been obtained for the first time in the present invention.

That is, the compounds of the general formula (I) exert a delayed antihypertensive effect in the case of elevated blood pressure by some cause (hypertension)

but produce no effect in normotensive cases, and, in addition, cause no acute hypotensive effect, thus having an excellent blood pressure-modulating action. Furthermore, it has been found that, since they do not cause evils common among conventional antihypertensives, i.e. severe side effects such as orthostatic hypotension, bradycardia or tachycardia, vomiting, nausea, headache and dizziness, they can be administered continuously for a long time period and, therefore, are very effective for treating hypertension.

Moreover, it has been demonstrated that the compounds exert an excellent pharmacological effect at a surprisingly small dose, i.e. a thousandth to a ten-thousandth of the dose for other antihypertensives. This finding and the aforementioned findings that the compounds are almost nontoxic and cause substantially no side effects have led to the conclusion that they are very convenient for use as compared with other antihypertensives.

Accordingly, the blood pressure-modulating agents according to the present invention, which contain the above-mentioned compounds (I) as an effective ingredient, greatly contribute as preventing agent and treating agent for hypertension at large.

Examples of the alkyl group having 1 to 15 carbon atoms represented by $R^1$, $R^2$ or $R^3$ in the above-mentioned general formula (I) include, for instance, methyl, ethyl, n-propyl, isopropyl, n-butyl, isobutyl, sec-butyl, tert-butyl, n-pentyl, isopentyl, sec-pentyl, neopentyl, tert-pentyl, n-hexyl, isohexyl, sec-hexyl, neohexyl, tert-hexyl, n-heptyl, 5-methylhexyl, octyl, nonyl, decyl, undecyl, dodecyl, tridecyl, tetradecyl and pentadecyl groups. As to the alkyl group having 1 to 15 carbon atoms represented by $R^4$, $R^5$, $R^6$, $R^7$, $R^8$ or $R^9$, there are exemplified such alkyl groups as described above. Examples of the halogen atom represented by $R^4$, $R^5$, $R^6$, $R^7$, $R^8$ or $R^9$ include, for instance, chlorine, bromine and iodine. Examples of X include, for instance,

halogen atoms such as chlorine, bromine and iodine, and the residual groups of perchloric acid, nitric acid and organic acids such as p-toluenesulfonic acid, nicotinic acid and orotic acid.

Among the compounds represented by the above-mentioned general formula (I), those wherein $R^4$, $R^5$ and $R^6$ are methyl group, and $R^7$, $R^8$ and $R^9$ are hydrogen atom are preferred. Among the preferred compounds, those wherein $R^1$, $R^2$ and $R^3$ are the same alkyl group, preferably heptyl group, more preferably n-heptyl group, are more preferred. In particular, platonin which is the compound wherein $R^1$, $R^2$ and $R^3$ are n-heptyl group, and X is iodine, that is, 4,4'-dimethyl-3,3'-di-n-heptyl-8-[2-(4-methyl-3-n-heptylthiazole]-2,2'-dicarbocyanine diiodide is most preferred.

The preferred indications of the blood pressure-modulating agent according to the present invention include a wide variety of hypertension, from mild to severe, such as malignant hypertension, essential hypertension, renal hypertension, neural hypertension, juvenile hypertension and endocrine hypertension.

The blood pressure-modulating agent of the invention exerts its effect to a satisfactory extent at a very slight adult-dose of about 10 to about 500 μg/one time (value calculated on the basis of the amount of the effective ingredient, hereinafter the same). In particular, a dose of 50 to 100 μg per 1 to 3 days is preferred.

The blood pressure-modulating agent of the present invention exerts its activity even when administered orally, and can be used in the form of tablet, capsule, powder, granule, liquid, etc. It can also be used in the form of collunarium, suppository, etc. Furthermore, it can also be administered transdermically in the form of patch. The blood pressure-modulating agent of the invention can be made into preparation forms in any usual manner using carriers usually employed without any particular restriction. In

incorporating the compounds (I) into the dosage forms mentioned above, the compounds (I) may also be used in the microencapsulated form.

The compounds (I) used in the invention can be prepared, for instance, in the following manners:

2,4-Dimethylthiazole (b.p. 146° to 147°C), which is readily synthesized from monochloroacetone and thioacetamide, is heated to react with an alkyl halide having 1 to 15 carbon atoms in an oil bath at about 170°C to give 3-alkyl-2,4-dimethylthiazolium halide. This compound is mixed with ethyl orthoformate and acetic anhydride and the resultant is subjected to reaction by heating at 140° to 145°C in an oil bath, and the obtained crude product is recrystallized from a solvent such as ethanol to give a desired compound, 4,4'-dimethyl-3,3'-dialkyl-8-[2-(4-methyl)-3-alkylthiazole]-2,2'-dicarbocyanine dihalide. The melting points of some of the compounds (I) obtained in this manner are shown in Table 1. Furthermore, employing platonin being the known compound as a starting material, a variety of compounds of the general formula (I) wherein X is other than iodine can be obtained. The melting points of the compounds (I) obtained in this manner are shown in Table 1. The compounds (I) in Table 1 are those wherein $R^4$, $R^5$ and $R^6$ are methyl group, and $R^7$, $R^8$ and $R^9$ are hydrogen atom.

Table 1

| Compound (I) | | | m.p. (°C) |
|---|---|---|---|
| No. | $R^1$, $R^2$, $R^3$ | X | |
| 1 | Methyl | I | 264 to 266 |
| 2 | n-Butyl | I | 219 to 222 |
| 3 | n-Octyl | I | 203 to 205 |
| 4 | n-Dodecyl | I | 198 to 200 |
| 5 | n-Heptyl | Cℓ | 150 to 155 |
| 6 | " | Br | 118 to 120 |
| 7 | " | $C\ell O_4$ | 210 to 213 |
| 8 | " | 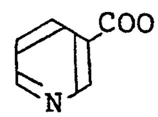 | 132 to 133 |
| 9 | " | 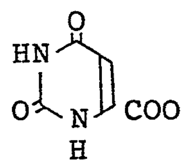 | 125 to 126 |
| Platonin | | | 202 to 204 |

BEST MODE FOR CARRYING OUT THE INVENTION

The blood pressure-modulating agent of the present invention will be explained by the following Examples. However, these Examples are intended to only illustrate the invention and the scope of the invention is not limited to those Examples.

Example 1

[Pharmacological experiments]

(1) Effect on spontaneously hypertensive rats

(Method)

Male spontaneously hypertensive rats (hereinafter referred to as SHR, weighing about 190 g) eight weeks old were used in groups of 5 animals. Platonin was orally adminstered at a dose of 1 μg/kg three times a week for 13 weeks. A physiological saline alone was adminstered to a control group in the same

manner as above. During the administration period, blood pressure, heart rate and body weight were measured at timed intervals.

Blood pressure, systolic, was bloodlessly measured in the caudal artery using a rat blood pressure manometer (made by Narco Biosystems Co.), and heart rate using a polygraph (made by Nippon Kohden Kabushiki Kaisha).

(Results)

(i) Effect on blood pressure

The results obtained are shown in Fig. 1. In Fig. 1 (also in Figs. 2 and 3), the platonin-given group is represented by a full line A, the saline-given group by a dotted line B and the administration period by C.

Platonin, when administered at a dose of 1 μg/kg three times a week, caused a remarkable blood pressure fall in SHR at each measurement time point after 5 weeks during the period of administration. In a separate experiment, though not illustrated here, platonin proved to have a characteristic such that the drug produces an antihypertensive effect very slowly, i.e., it shows no effect at all till the 3rd week of administration but causes a significant decrease in blood pressure after about 4 weeks of administration. When platonin was administered at a higher dose, the time required for the antihypertensive effect to be revealed was the same. Thus, any acute hypotensive effect was not found.

(ii) Effect on heart rate

At any time point during the period of administration of platonin, any significant change in heart rate was not observed, as compared with the control group.

(iii) Body weight change

The results are shown in Fig. 2. Platonin, when administered orally at a dose of 1 μg/kg three times a week, cuased no influence on body weight gain in SHR, indicating that the drug is safe. Though not illustrated

in the drawings, the drug, when administered orally at a dose of 1 to 100 μg/kg three times a week, produced no effect on body weight.

(2) Effect on blood pressure in normal rats

(Method)

Male Wistar-Kyoto rats eight weeks old (weighing about 190 g) were used in groups of 5 animals. Platonin was orally administered at a dose of 1 μg/kg three times a week for 8 weeks. A physiological saline alone was administered to a control group in the same manner as above. During the administration period, blood pressure, heart rate and body weight were measured at timed intervals. Blood pressure, systolic, was bloodlessly measured in the caudal artery using a rat blood pressure manometer (made by Narco Biosystems Co.), and heart rate using a polygraph (made by Nippon Kohden Kabushiki Kaisha).

(Results)

The results are shown in Fig. 3. It was found that platonin, when administered at a dose of 1 μg/kg three times a week, caused no influence on blood pressure in normal rats (Wistar-Kyoto) as in the control group.

Platonin, in the dose range of 1 to 100 μg/kg, caused no change in blood pressure in normal rats, either. In comparison with the control group, no significant changes in heart rate and body weight were observed.

(3) Acute toxicity

Eight male Wistar rats (weighing about 150 g) per group were used. Platonin suspended in 5 % gum arabic was administered intraperitonealy, or orally using a gastric tube. The animals were observed for death for 7 days. The $LD_{50}$ value was calculated by the method of Van der Wärden. The intraperitoneal $LD_{50}$ value was 54 mg/kg and the oral $LD_{50}$ value 1.5 g/kg. This finding, taken in conjunction with the aforementioned result that the drug is effective in antihypertensive effect at a dose of 1 μg/kg, indicates that the drug is a compound having a noticeably wide safety margin.

0144441

In addition, it is confirmed that the compounds (I) other than platonin also exert effects on the same level as those of platonin in the same experiments as in the above.

Example 2

Employing platonin and Compound Nos. 1 to 9 as an effective ingredient, respectively, there were prepared blood pressure-modulating agents in a variety of preparation forms as in the followings:

(1) Tablet

Tablets having the following composition were prepared in a usual manner.

| Component | mg/tablet |
|---|---|
| Effective ingredient | 0.05 |
| Lactose | 79.95 |
| Corn starch | 62.50 |
| Sucrose fatty acid ester | 7.50 |
| Total | 150 |

In case of gastric coated tablets, the above tablets were further subjected to a 5 % by weight coating of Tc-5, followed by application of a sugar coating. In case of enteric coated tablets, the above tablets were further subjected to a 10 % by weight coating of HP-55, followed by application of a sugar coating.

(2) Capsule

Capsules having the following composition were prepared in a usual manner.

| Component | mg/capsule |
|---|---|
| Effective ingredient | 0.05 |
| Lactose | 146.95 |
| Sucrose fatty acid ester | 3.00 |
| Total | 150 |

0144441

(3) Powder

A powder having the following composition was prepared in a usual manner.

| Component | mg |
|---|---|
| Effective ingredient | 0.05 |
| Lactose | 499.95 |
| Total | 500 |

(4) Suppository

Two kinds of suppositories having the following compositions were prepared in a usual manner.

Suppository A

| Component | mg/suppository |
|---|---|
| Effective ingredient | 0.05 |
| PEG #1000 | 1,440 |
| PEG #4000 | 59.95 |
| Total | 1,500 |

Suppository B

| Component | mg/suppository |
|---|---|
| Effective ingredient | 0.05 |
| Witepsol H-15 | 1,280 |
| Witepsol E-85 | 319.95 |
| Total | 1,600 |

(5) Syrup

A syrup (dosage/one time: 50 μg/5 ml) having the following composition was prepared in a usual manner.

| Component | Content/100 ml |
|---|---|
| Effective ingredient | 1 mg |
| Sugar | 60 g |
| Glycerin | 10 g |
| Sodium citrate | 0.1 g |
| Sodium benzoate | 0.3 g |
| Saccharin sodium salt | 0.1 g |
| Purified water | Appropriate amount |

(6) Patch

A patch having the following composition was prepared in a usual manner.

| Component | mg |
|---|---|
| Effective ingredient | 0.05 |
| Adhesive containing polybutene as a main component | 1000 |

CLAIMS

1. A blood pressure-modulating agent which contains as an effective ingredient a trithiazole pentamethine cyanine compound represented by the general formula (I):

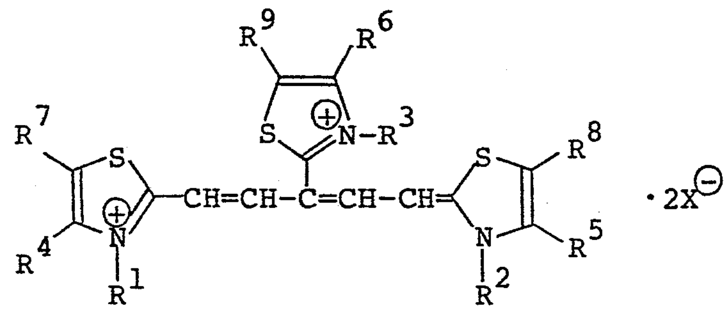

(I)

wherein $R^1$, $R^2$ and $R^3$ are the same or different and each is an alkyl group having 1 to 15 carbon atoms, $R^4$, $R^5$, $R^6$, $R^7$, $R^8$ and $R^9$ are the same or different and each is hydrogen atom, an alkyl group having 1 to 15 carbon atoms, hydroxyl group or a halogen atom, and X is a halogen atom, the residual group of perchloric acid, the residual group of nitric acid or the residual group of an organic acid.

2. The blood pressure-modulating agent of Claim 1, wherein $R^4$, $R^5$ and $R^6$ are methyl group, and $R^7$, $R^8$ and $R^9$ are hydrogen atom.

3. The blood pressure-modulating agent of Claim 2, wherein $R^1$, $R^2$ and $R^3$ are n-heptyl group, and X is iodine.

4. The blood pressure-modulating agent of Claim 1, which is in a preparation form for oral administration.

FIG. 1

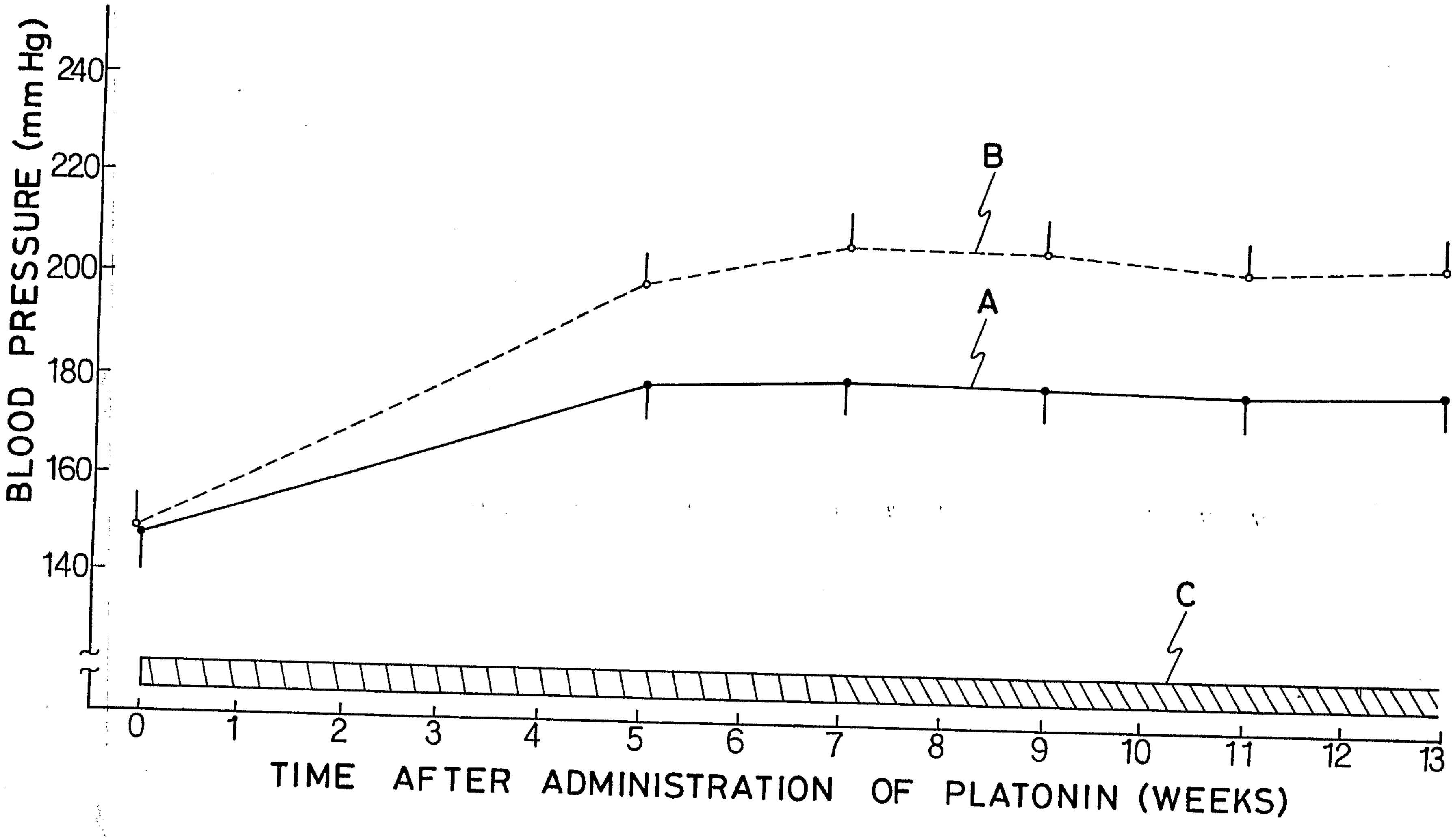
BLOOD PRESSURE (mm Hg)
240
220
200
180
160
140
B
A
C
0
1
2
3
4
5
6
7
8
9
10
11
12
13
TIME AFTER ADMINISTRATION OF PLATONIN (WEEKS)

FIG. 2

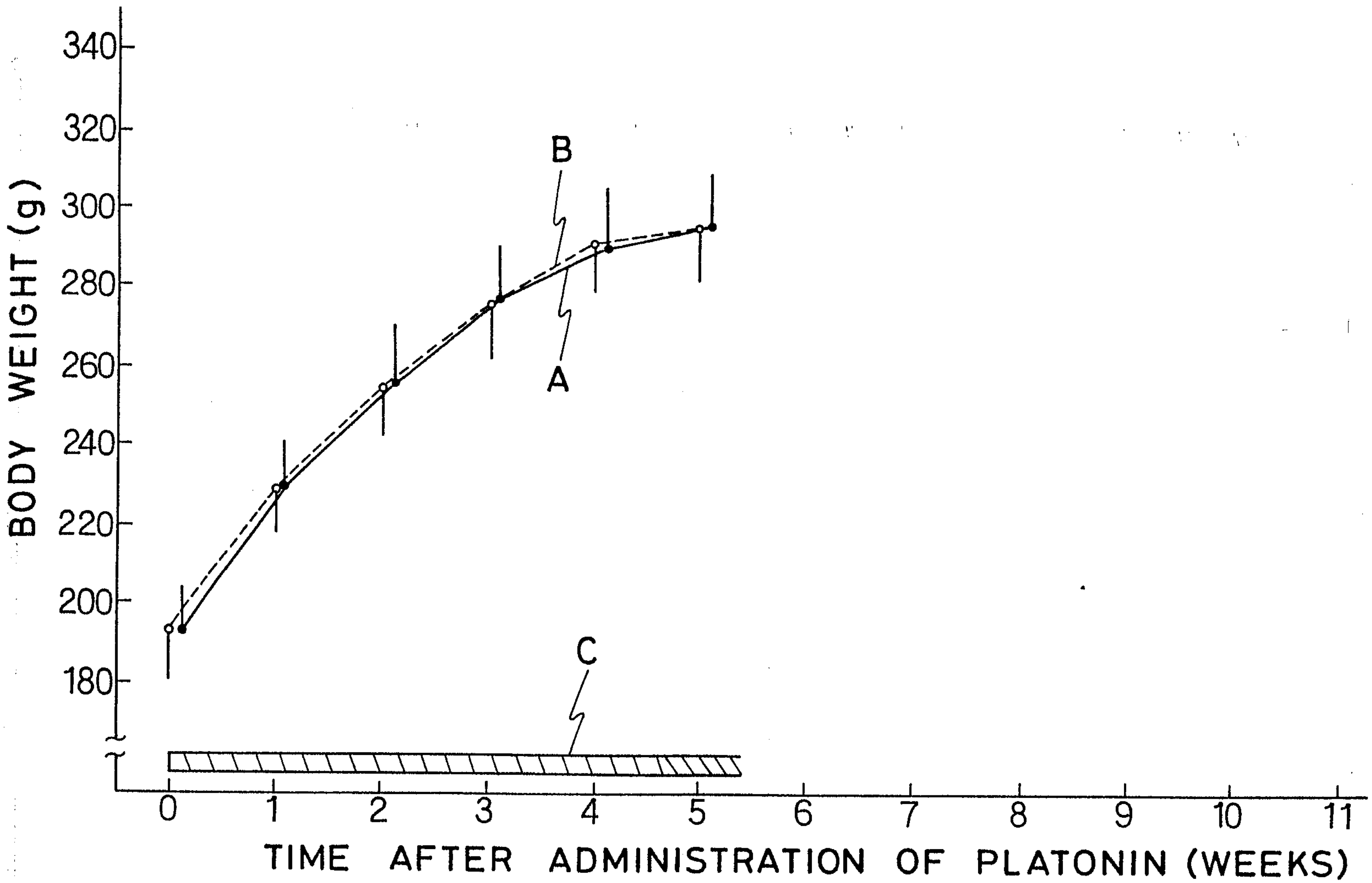
BODY WEIGHT (g)
340
320
300
280
260
240
220
200
180
B
A
C
0
1
2
3
4
5
6
7
8
9
10
11
TIME AFTER ADMINISTRATION OF PLATONIN (WEEKS)

FIG. 3

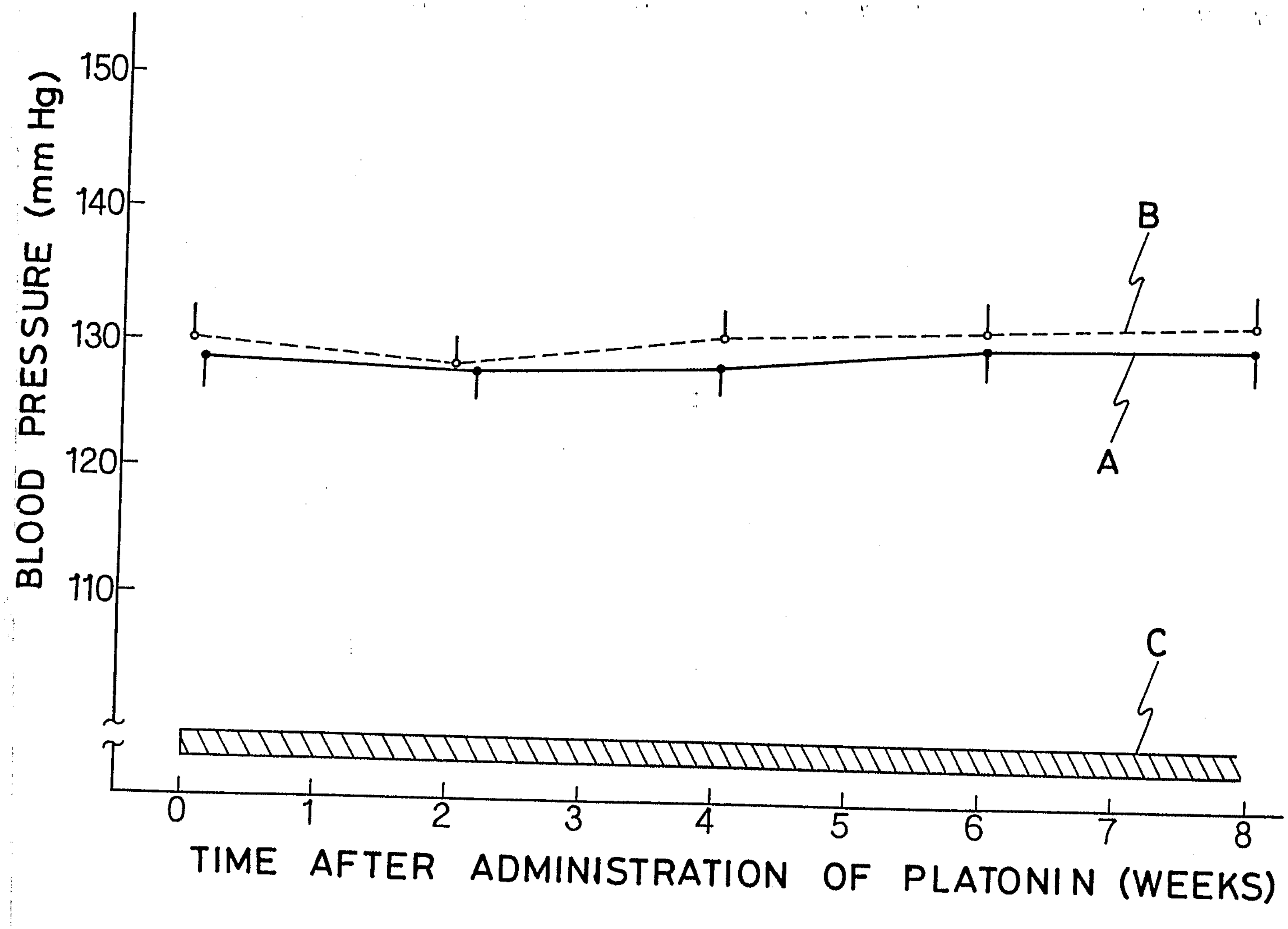
BLOOD PRESSURE (mm Hg)
150
140
130
120
110
B
A
C
0
1
2
3
4
5
6
7
8
TIME AFTER ADMINISTRATION OF PLATONIN (WEEKS)

0144441

# INTERNATIONAL SEARCH REPORT

International Application No. PCT/JP84/00255

**I. CLASSIFICATION OF SUBJECT MATTER** (if several classification symbols apply, indicate all) [3]

According to International Patent Classification (IPC) or to both National Classification and IPC

Int. $Cl^3$ A61K31/425

**II. FIELDS SEARCHED**

Minimum Documentation Searched [4]

| Classification System | Classification Symbols |
|---|---|
| IPC | A61K 31/425<br>C07D 277/22 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| X | JP, B1, 43-3884 (Nippon Kanko Shikiso Kenkyusho Kabushiki Kaisha)<br>13 February 1968 (13. 02. 68) | 1 - 4 |
| X | JP, B1, 43-3885 (Nippon Kanko Shikiso Kenkyusho Kabushiki Kaisha)<br>13 February 1968 (13. 02. 68) | 1 - 4 |
| X | See Chemical Abstracts Vol. 76, No. 19 (08. 05. 72) (Columbus, Ohio, U.S.A.) Suzue, S., "Effect of photosensitizing dyes on blood pressure" 3rd and 5th Reports, Page 56, 1st Step, Abstract No. 76, 108237r and 76, 108239t, Kanko Shikiso 1971, No. 80, and No. 80, 25 | 1 - 4 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| August 7, 1984 (07. 08. 84) | August 20, 1984 (20. 08. 84) |
| International Searching Authority [1] | Signature of Authorized Officer [20] |
| Japanese Patent Office | |